# FASCICULE DE BREVET EUROPEEN

(11) **EP 1 446 064 B1**
(45) Date de publication et mention de la délivrance du brevet: **20.06.2007**
(21) Numéro de dépôt: 02760383.6
(22) Date de dépôt: 08.07.2002
(51) Int. Cl.: A61B 19/00

(54) **DISPOSITIF DE ROBOT D'ASSISTANCE AU GUIDAGE D'INSTRUMENTS CHIRURGICAUX POUR LE TRAITEMENT DE LA MATIERE OSSEUSE**
ROBOTERVORRICHTUNG ZUR NACHFÜHRUNG EINES CHIRURGISCHEN INSTRUMENTES ZUR BEHANDLUNG VON KNOCHEN
SURGICAL INSTRUMENT GUIDING AID ROBOT DEVICE FOR TREATING BONE MATTER

(30) Priorité: 03.09.2001 FR 0111361
(43) Date de publication de la demande: 18.08.2004
(73) Titulaire: Zimmer GmbH, 8404 Winterthur (CH)
(72) Inventeur: NAHUM, Bertin, 31100 Toulouse (FR)
(74) Mandataire: Manitz, Finsterwald & Partner GbR
(86) Numéro de dépôt international: PCT/FR2002/002381
(87) Numéro de publication internationale: WO 2003/020147

(56) Documents cités:
- WO-A-99/37220
- US-A- 5 828 813
- US-B1- 6 197 017
- US-B1- 6 233 504

## Description

La présente invention a pour objet dans le domaine de la chirurgie un dispositif de robot d'assistance au guidage d'instruments chirurgicaux pour le traitement de la matière osseuse.

De nombreuses spécialités chirurgicales impliquent dans certains cas la découpe, le fraisage ou l'alésage précis de tissu osseux.
C'est par exemple le cas lors de poses de certaines prothèses, de craniotomies ou de décompressions discales.

Ces procédures sont traditionnellement effectuées à l'aide d'instruments le plus souvent motorisés tels que perceuse, fraiseuse, scie, etc. qui sont manuellement positionnés, dirigés et maintenus par le chirurgien. La précision avec laquelle la cavité osseuse est réalisée ainsi que sa qualité sont en conséquences dépendantes de la dextérité du chirurgien et de sa capacité à diriger et maintenir précisément ses instruments dans les positions et directions voulues. Cela, en dépit de la forme ou de l'état de surface de l'os favorisant parfois des dérapages des instruments.

On connaît déjà des dispositifs d'assistance, permettant de guider le geste du chirurgien.

Le document US 5,828,813 décrit un robot d'assistance au guidage d'instruments chirurgicaux, comportant un bras articulé destiné à être manoeuvrée manuellement par le chirurgien et équipé de moyens qui, en association avec les moyens moteurs dudit bras motorisé, permettent que ce dernier reproduise tous les mouvements dudit bras articulé. Le préambule de la revendication 1 est basé sur ce document.

Un autre exemple est décrit dans le document US 5.299.288 qui consiste en un dispositif robotisé utilisé pour la réalisation d'un fraisage optimum de la cavité fémorale en vue de la pose d'une prothèse de la hanche. Ce dispositif robotisé est du type dit "guidés par l'image", en ce sens qu'il nécessite l'utilisation d'images numériques de la zone osseuse à traiter, obtenues au moyen d'un scanner, et à partir desquelles la tâche dudit dispositif est programmée par l'intermédiaire d'un logiciel de Planification. Cette manière de procéder nécessite des opérations préalables de calibrage, en vue de mettre en correspondance le référentiel image et le référentiel patient.

Un tel dispositif présente donc l'inconvénient de nécessiter préalablement à l'intervention une programmation et une reconnaissance numérique au moyen d'un scanner. Ainsi, chaque type d'intervention nécessite un logiciel spécifique.

On connaît également des dispositifs de robot dits "semi actifs ", où l'instrument chirurgical est porté à l'extrémité d'un bras articulé mais est actionné manuellement par le chirurgien, ledit bras articulé permettant, par l'intermédiaire d'une pré programmation du type de celle décrite précédemment c'est-à-dire comprenant un logiciel de planification, de limiter la zone d'action dudit instrument. Les gestes du chirurgien sont enregistrés grâce à un capteur de force situé au niveau de liaison avec l'instrument chirurgical, et tant que ce dernier reste à l'intérieur de la zone pré programmée, le contrôleur du robot génère automatiquement des consignes de déplacement de façon à accompagner le geste.

Un tel dispositif présente le même inconvénient que celui des dispositifs du type dit "guidés par l'image", en ce sens qu'ils nécessitent une planification à partir d'images préopératoires.

On connaît également un dispositif de robot dit semi actif décrit dans le document WO 99/37220, et qui consiste en un appareil de guidage d'un instrument de chirurgie monté sur un mécanisme relié à la tête d'une unité de positionnement robotisée. Ledit mécanisme crée un plan de déplacement partie intégrante de la tête à plusieurs degrés de liberté recourant à des bras tournant les uns par rapport aux autres, tandis que l'unité de positionnement, reliée à une embase mobile sur des roues placée sous la table d'opération et pouvant lui être fixée, amène la tête en un point donné de l'espace, ce qui permet de placer l'instrument à proximité du site de l'opération. Le plan du mécanisme partie intégrante de la tête, étant parallèle au plan d'une surface de référence à créer dans l'os, l'instrument à main découpe avec précision ladite surface de référence parallèlement audit plan.

Ce dispositif présente le même inconvénient que celui précité pour les dispositifs de robot dits semi-actifs, par ailleurs son utilisation est plus restrictive puisque le geste du chirurgien est limité dans un plan.

La présente invention a pour but de proposer un dispositif de robot d'assistance au guidage d'instruments chirurgicaux pour le traitement de la matière osseuse permettant de remédier aux divers inconvénients précités.

Le dispositif de robot d'assistance au guidage d'instruments chirurgicaux pour le traitement de la matière osseuse selon la présente invention est du type comportant un bras robotisé à au moins cinq degrés de liberté et des moyens moteurs, apte à porter en extrémité un instrument chirurgical, ce dispositif comportant un système de commande qui consiste en un bras articulé à au moins six degrés de liberté, dont l'extrémité libre est destinée à être manoeuvrée manuellement par le chirurgien, le bras articulé étant équipé de moyens qui, en association avec les moyens moteurs dudit bras robotisé, permettent que le bras robotisé reproduise les mouvements dudit bras articulé, le dispositif comportant des moyens pour enregistrer des positions extrêmes autorisées du bras robotisé déterminées préalablement à l'intervention chirurgicale par le pré-positionnement de l'instrument chirurgical sur le contour de l'enveloppe de travail voulue et pour définir un volume spatial de travail du bras robotisé, et en ce que le champ d'action de l'instrument chirurgical est limité audit volume spatial de travail lors de l'intervention chirurgicale.

Le dispositif selon l'invention permet de sécuriser le geste chirurgical en offrant la possibilité de limiter au préalable l'enveloppe de travail dans laquelle l'instrument chirurgical va évoluer. Il est de plus d'une mise en oeuvre aisée et rapide.

Par ailleurs, le dispositif selon l'invention s'intègre facilement dans l'environnement du bloc opératoire et est compatible avec les contraintes qui lui sont propres.

De préférence, le bras robotisé est mobile en déplacement et est équipé d'un système d'ancrage permettant une installation simple et stable au niveau de la table d'opération.

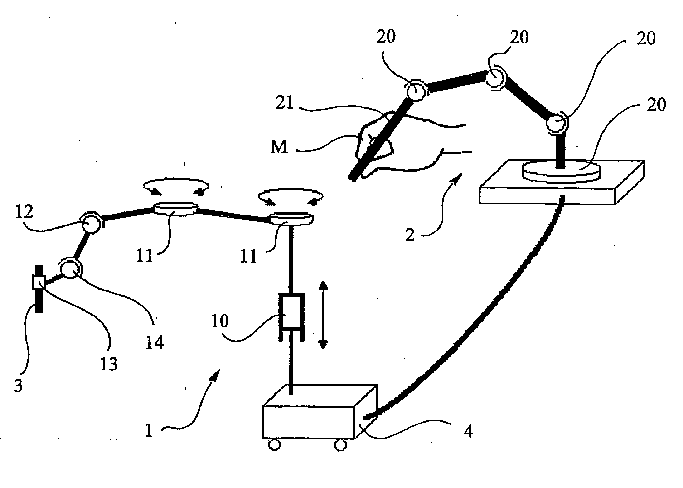

Les avantages et les caractéristiques du dispositif selon l'invention, ressortiront plus clairement de la description qui suit et qui se rapporte au dessin annexé, lequel en représente un mode de réalisation non limitatif.

Dans le dessin annexé, la figure unique représente une vue schématique en perspective d'un dispositif de robot d'assistance au guidage d'instruments chirurgicaux pour le traitement de la matière osseuse selon l'invention.

En référence à cette figure, on peut voir qu'un dispositif de robot d'assistance au guidage d'instruments chirurgicaux pour le traitement de la matière osseuse selon l'invention comporte un bras robotisé 1 commandé par l'intermédiaire d'un bras articulé 2 apte à être manoeuvré manuellement par le chirurgien dont est représentée la main M.

Le bras robotisé 1 est de préférence à au moins cinq degrés de liberté par l'intermédiaire d'articulations motorisées, en l'occurrence il comprend un actionneur prismatique 10, deux liaisons rotoïdes 11, et un poignet 12, et son extrémité libre est munie d'un porte-outil 13 destiné à la préhension d'un instrument chirurgical 3, tel qu'une fraiseuse, une perceuse ou une scie.

On notera que le bras articulé comporte de plus un orienteur 14 interposé entre le porte outil 13 et le reste du bras robotisé 1, en sorte de permettre un pré-positionnement de l'instrument 3.

Le bras robotisé 1 est porté par une plate-forme mobile 4 permettant son déplacement, sachant que celle-ci et/ou le bras robotisé 1 est muni de moyens d'ancrage, non représentés, permettant un arrimage à la table d'opération.

Le bras articulé 2 est à au moins six degrés de liberté, il comporte des articulations 20 permettant que son extrémité libre 21 puisse être manoeuvrée par le chirurgien comme si celui-ci manipulait l'instrument chirurgical. Il est par ailleurs muni, au niveau de chacune des articulations 20, de capteurs, non représentés, permettant de mesurer tous les mouvements exécutés par le chirurgien.

Les mouvements mesurés par les capteurs sont transmis au bras robotisé 1 qui les reproduit servilement et déplace de la même manière l'instrument chirurgical 3.

Dans un but de sécurisation du geste du chirurgien, le dispositif de robot d'assistance au guidage d'instruments chirurgicaux selon l'invention, est équipé d'un système permettant de limiter le champ d'action de l'instrument 3, sans avoir recourt à un logiciel de planification comme c'est le cas des dispositifs existants.

A cet effet, les articulations motorisées du bras robotisé 1 sont aptes à enregistrer des positions extrêmes autorisées lesquelles sont déterminées préalablement à l'intervention chirurgicale par une routine de définition du volume spatial de travail.

Cette routine consiste à déterminer les contours d'une zone à l'intérieur de laquelle l'instrument chirurgical 3 devra évoluer.

Pour cela, le chirurgien déplace successivement l'instrument chirurgical 3 sur des points décrivant le contour de l'enveloppe de travail voulue, en sorte de réaliser l'enregistrement de chacune de ces positions. Lors de l'intervention chirurgicale, la partie active de l'instrument 3 évoluera uniquement dans l'espace délimité préalablement, sans répercuter d'éventuels déplacements hors limites transmis accidentellement au bras articulé 2.

Le dispositif selon l'invention peut être utilisé rapidement et aisément quel que soit le type d'intervention sur la matière osseuse considérée, contrairement aux dispositifs existants qui nécessitent préalablement une numérisation au moyen d'un scanner de la zone osseuse à traiter, et la programmation d'un logiciel de planification.

## Revendications

1. Dispositif de robot d'assistance au guidage d'instruments chirurgicaux pour le traitement de la matière osseuse, du type comportant un bras robotisé (1) à au moins cinq degrés de liberté, apte à porter en extrémité un instrument chirurgical (3) et des moyens moteurs (10, 11, 12, 14), ce dispositif comportant un système de commande qui consiste en un bras articulé (2) à au moins six degrés de liberté, dont l'extrémité libre (21) est destinée à être manoeuvrée manuellement par le chirurgien, ledit bras articulé étant équipé de moyens (20) qui, en association avec les moyens moteurs dudit bras robotisé, permettent que le bras robotisé reproduise les mouvements dudit bras articulé, **caractérisé en ce que** lesdits moyens moteurs sont associés à des moyens aptes pour enregistrer les positions extrêmes autorisées du bras robotisé déterminées préalablement à l'intervention chirurgicale par le pré-positionnement dudit instrument chirurgical (3) sur le contour de l'enveloppe de travail voulue et pour définir un volume spatial de travail du bras robotisé, et **en ce que** le champ d'action de l'instrument chirurgical (3) est limité audit volume spatial de travail lors de l'intervention chirurgicale.

2. Dispositif selon la revendication 1, **caractérisé en ce que** le bras robotisé (1) comprend un actionneur prismatique (10), deux liaisons rotoïdes (11) et un poignet (12), tandis que son extrémité libre est munie d'un porte outil (13) destiné à la préhension d'un instrument chirurgical (3).

3. Dispositif selon l'une quelconque des revendications 1 ou 2, **caractérisé en ce que** le bras robotisé (1) est porté par une plate forme mobile (4) permettant son déplacement.

4. Dispositif selon l'une quelconque des revendications 1 à 3, **caractérisé en ce qu'**il comporte des moyens d'ancrage permettent un arrimage du bras robotisé à la table d'opération.

5. Dispositif selon l'une quelconque des revendications 1 à 4, **caractérisé en ce que** le bras articulé (2) comporte, au niveau de chacune de ses articulations, des capteurs adaptés à mesurer les mouvements exécutés par la main du chirurgien.

6. Dispositif selon l'une quelconque des revendications 1 à 5, **caractérisé en ce que** le bras robotisé (1) comporte des articulations aptes à enregistrer des positions extrêmes autorisées.

## Claims

1. Robot device for assisting the guidance of surgical instruments for treating bone matter, of the type comprising a robotic arm (1), which has at least five degrees of freedom and is capable of carrying a surgical instrument (3) at the end thereof, and drive means (10, 11, 12, 14), this device comprising a control system consisting of an articulated arm (2) which has at least six degrees of freedom and the free end (21) of which is intended to be manoeuvred manually by the surgeon, said articulated arm being equipped with means (20) which, together with said robotic arm drive means, enable the robotic arm to reproduce the movements of said articulated arm, **characterised in that** said drive means are connected to means for recording the permitted end positions of the robotic arm, which positions are determined prior to the surgical operation by pre-positioning said surgical instrument (3) on the contour of the desired working envelope, and for defining a spatial working volume for the robotic arm, and **in that** the field of action of the surgical instrument (3) is limited to said spatial working volume during the surgical operation.

2. Device according to claim 1, **characterised in that** the robotic arm (1) comprises a prismatic actuator (10), two rotary joints (11) and a wrist (12), while its free end is equipped with a tool holder (13) intended for gripping a surgical instrument (3).

3. Device according to either claim 1 or claim 2, **characterised in that** the robotic arm (1) is carried by a movable platform (4) which allows the displacement thereof.

4. Device according to any one of claims 1 to 3, **characterised in that** it comprises anchoring means for securing the robotic arm to the operating table.

5. Device according to any one of claims 1 to 4, **characterised in that** the articulated arm (2) comprises, in the region of each of its joints, sensors adapted to measure the movements performed by the surgeon's hand.

6. Device according to any one of claims 1 to 5, **characterised in that** the robotic arm (1) comprises joints capable of recording permitted end positions.

## Patentansprüche

1. Robotervorrichtung zur gestützten Führung von chirurgischen Instrumenten für die Behandlung von Knochenmaterial, umfassend einen robotisierten Arm (1) mit mindestens fünf Freiheitsgraden, der in der Lage ist, am Ende ein chirurgisches Instrument (3) zu tragen, und Antriebsmittel (10, 11, 12, 14), wobei diese Vorrichtung ein Steuersystem umfasst, das in einem gelenkigen Arm (2) mit mindestens sechs Freiheitsgraden besteht, dessen freies Ende (21) dazu bestimmt ist, durch den Chirurgen manuell manövriert zu werden, wobei dieser gelenkige Arm mit Mitteln (20) ausgerüstet ist, die in Kombination mit den Antriebsmitteln des robotisierten Arms gestatten, dass der robotisierte Arm die Bewegungen des gelenkigen Arms reproduziert, **dadurch gekennzeichnet, dass** die Antriebsmittel mit Mitteln kombiniert sind, die in der Lage sind, die zugelassenen Endstellungen des robotisierten Arms zu registrieren, die vor dem chirurgischen Eingriff durch die Vorpositionierung des chirurgischen Instruments (3) auf dem Umriss der gewünschten Arbeitseinhüllenden bestimmt werden, und ein räumliches Arbeitsvolumen des robotisierten Arms zu definieren, und dass das Aktionsfeld des chirurgischen Instruments (3) bei dem chirurgischen Eingriff auf dieses räumliche Arbeitsvolumen begrenzt ist.

2. Vorrichtung nach Anspruch 1, **dadurch gekennzeichnet, dass** der robotisierte Arm (1) ein prismenförmiges Stellglied (10), zwei Rotoidverbindungen (11) und einen Griff (12) umfasst, während sein freies Ende mit einem Werkzeughalter (13) versehen ist, der zum Ergreifen eines chirurgischen Instruments (3) bestimmt ist.

3. Vorrichtung nach einem der Ansprüche 1 oder 2, **dadurch gekennzeichnet, dass** der robotisierte Arm (1) von einer beweglichen Plattform (4) getragen ist, die seine Bewegung gestattet.

4. Vorrichtung nach einem der Ansprüche 1 bis 3, **dadurch gekennzeichnet, dass** sie Verankerungsmittel umfasst, die ein Festmachen des robotisierten Arms an dem Operationstisch gestatten.

5. Vorrichtung nach einem der Ansprüche 1 bis 4, **dadurch gekennzeichnet, dass** der gelenkige Arm (2) auf Höhe jedes seiner Gelenke Fühler umfasst, die dafür ausgelegt sind, die von der Hand des Chirurgen ausgeführten Bewegungen zu messen.

6. Vorrichtung nach einem der Ansprüche 1 bis 5, **dadurch gekennzeichnet, dass** der robotisierte Arm (1) Gelenke umfasst, die in der Lage sind, zugelassene Endstellungen zu registrieren.
